# EUROPÄISCHE PATENTSCHRIFT

(11) **EP 1 663 031 B1**
(45) Veröffentlichungstag und Bekanntmachung des Hinweises auf die Patenterteilung: **10.09.2008**
(21) Anmeldenummer: 03818483.4
(22) Anmeldetag: 08.09.2003
(51) Int. Cl.: A61B 17/70

(54) **LÄNGSTRÄGER**
LONGITUDINAL SUPPORT
LONGERON

(43) Veröffentlichungstag der Anmeldung: 07.06.2006
(73) Patentinhaber: Synthes GmbH, 4436 Oberdorf (CH)
(72) Erfinder: HARTMANN, Stephan, CH-4500 Solothurn (CH); STUDER, Armin, CH-4513 Langendorf (CH)
(74) Vertreter: Lusuardi, Werther
(86) Internationale Anmeldenummer: PCT/CH2003/000605
(87) Internationale Veröffentlichungsnummer: WO 2005/023125

(56) Entgegenhaltungen:
- EP-A- 1 064 885
- WO-A-03/068088
- DE-U- 9 004 960
- DE-U- 9 402 695
- US-A- 5 520 689
- US-B1- 6 296 644

## Beschreibung

Die Erfindung bezieht sich auf eine Vorrichtung zur Stabilisierung von Wirbelkörpern, gemäss dem Patentanspruch 1.

Die gegenwärtige Behandlung bei beschädigten oder tumorbehafteten Wirbelkörpern beinhaltet üblicherweise die Implantation eines starren, mittels Knochenverankerungsmittel an den Wirbelkörpern verankerten Längsträgers oder einer starren Knochenplatte. Diese Vorrichtungen bezwecken, durch die starren Implantate die Bewegung der auf diese Weise stabilisierten Wirbelkörper relativ zueinander zu verhindern und die Fusion der benachbarten Wirbelkörper zu fördern.

Eine Vorrichtung zur Stabilisierung von Wirbelkörpern mit einem multisegmentalen, in einer gewünschten Form fixierbaren Längsträger ist aus der FR 2 796 828 JAMMET bekannt. Damit lässt sich der aus mehreren axialen Segmenten bestehende Längsträger mit den Kopfsegmenten der für die Stabilisierung vorgesehenen, in die Pedikel der entsprechenden Wirbelkörper eingeschraubten Pedikelschrauben oder - haken verbinden und ohne grosse Kraftaufwendung an die durch die Positionen der Pedikelschrauben vorgegebene Form anpassen. Nachteilig an dieser bekannten Vorrichtung ist, dass die Längsträgerelemente einzeln an den Kopfsegmenten der Pedikelschrauben oder Pedikelhaken befestigbar und nicht miteinander verbindbar sind, so dass jedes Längsträgerelement einzeln in den Körper des Patienten eingeführt und plaziert werden muss, was für den Operateur einen mühsamen und zeitraubenden Operationsschritt darstellen kann.

Eine Vorrichtung gemäss dem Oberbegriff des Anspruchs 1 ist aus DE 94 02 695 U bekannt.

Hier will die Erfindung Abhilfe schaffen. Der Erfindung liegt die Aufgabe zugrunde, ein Längsträgerelement mit einer Gelenkschale zu schaffen, in deren Kavität ein komplementärer Gelenkkkopf eines weiteren Längsträgerelementes elastisch einschnappbar ist, so dass ein aus mehreren Längsträgerelementen bestehender Längsträger ausserhalb des Körpers des Patienten vormontierbar ist.

Die Erfindung löst die gestellte Aufgabe mit einer Vorrichtung zur Stabilisierung von Wirbelkörpern, welche die Merkmale des Anspruchs 1 aufweist.

Die durch das Längsträgerelement erreichten Vorteile sind im wesentlichen darin zu sehen, dass
- ein aus mehreren miteinander verbindbaren Längsträgerelementen bestehender Längsträger vormontierbar ist;
- der Längsträger in-situ ohne Kraftaufwand an die gewünschte Geometrie anpassbar ist; und
- die Implantation des Längsträgers erheblich vereinfacht werden kann.

Die Gelenkschale kann in einer bevorzugten Ausführungsform aus einer Nickel-Titanlegierung (Nitinol) bestehen, wobei 45 % < Ni < 55 %, 45 % < Ti < 55 % und x + y = 100 % ist. Ein solches Material ist besonders bioverträglich und hochelastisch.

In einer anderen Ausführungsform ist die Gelenkschale aussen sphärisch konvex ausgebildet, wodurch der Vorteil einer polyaxial schwenkbaren Aufnahme der Gelenkschale beispielsweise in einem als Tulpenschraube ausgebildeten Knochenverankerungsmittel erreichbar ist.

In wiederum einer anderen Ausführungsform umfasst das erste Verbindungssegment des Längsträgerelementes ein Aussengewinde.

Die Vorteile des Längsträgers sind im wesentlichen darin zu sehen,dass
- eine Gelenkschale aufweisende, erste Längsträgerelemente polyaxial schwenkbar mit zweiten, einen komplementären Gelenkkopf aufweisenden Längsträgerelementen zusammenfügbar sind; und
- der Längsträger der Anatomie entsprechend formbar ist.

In einer bevorzugten Ausführungsform ist die Verbindung zwischen einem ersten Verbindungssegment eines ersten Längsträgerelementes und einem ersten Verbindungssegment eines zweiten Längsträgerelementes axial verlänger- respektive verkürzbar, so dass ein erstes Längsträgerelement koaxial mit einem zweiten Längsträgerelement zusammenfügbar sind und das durch die zwei Längsträgerelemente gebildete Längsträgersegment längenverstellbar ist.

Diese längenverstellbare Verbindung zwischen den zwei Längsträgerelementen kann teleskopierbar sein oder als Schraubverbindung realisiert sein. Dazu kann an ersten Längsträgerelement das erste Verbindungssegment als koaxialer Schaft ausgebildet sein und das Gewinde als Aussengewinde ausgestaltet sein und am zweiten Längsträgerelement das erste Verbindungssegment als Hülse ausgebildet sein, welche eine Zentralbohrung mit einem zum Aussengewinde komplementären Innengewinde umfasst.

In einer weiteren Ausführungsform ist der Gelenkkopf sphärisch konvex und die Kavität der Gelenkschale komplementär ausgestaltet.

In einer anderen Ausführungsform sind der Gelenkkopf und die Gelenkschale elastisch ineinander einschnappbar. Dadurch ist der Vorteil erreichbar, dass eine einfache Vormontage eines aus mehreren Längsträgersegmenten bestehenden Längsträgers möglich ist.

In wiederum einer anderen Ausführungsform umfasst der Längsträger ein Endstück mit einem in eine Gelenkschale einschnappbaren Gelenkkopf. Dadurch ist der Vorteil erreichbar, dass ein endständig am Längsträger angeordnete Gelenkschale bei ihrer Montage in die Aufnahme am Kopf einer Pedikelschraube nicht zusammengepresst wird. Vorzugsweise umfasst der Längsträger ein zweites Endstück mit einer Gelenkschale umfasst.

Die erfindungsgemässe Vorrichtung zur Stabilisierung von Wirbelkörpern umfasst einen Längsträger gemäss einer der oben dargestellten Ausführungsformen und
A) mindestens zwei Knochenverankerungsmittel mit je einer Zentralachse, je einem Verankerungssegment zur Fixierung des Knochenverankerungsmittels an einem Knochen, insbesondere an einem Wirbelkörper und je einem Kopfsegment mit Mitteln zur Aufnahme der Gelenkschale; und
B) Fixationsmitteln zur Fixierung der Gelenkschalen in den Mitteln zur Aufnahme der Gelenkschale.

In einer bevorzugten Ausführungsform sind die Mittel zur Aufnahme der Gelenkschale eine konkave, zur Gelenkschale komplementär sphärische Ausnehmung ausgestaltet.

Die Fixationsmittel umfassen eine elastisch spreizbare Klammer, welche über die Gelenkschale bringbar und am Kopfsegment des Knochenfixationsmittels einschnappbar ist, wobei die Gelenkschale nach Montage der Klammer durch diese zusammengepresst wird. Damit ist der Vorteil erreichbar, dass durch das Zusammenpressen des zweiten Gelenkteiles einerseits das Gelenk zwischen den beiden Längsträgersegmenten blockiert und andererseits der Längsträger an diesem Knochenverankerungsmittel fixiert wird.

Vorzugsweise hat die Klammer an ihren freien Enden je einen Nocken, welcher am Kopfsegment des Knochenverankerungsmittels einrastbar ist. Zur besseren Aufnahme der Nocken können am Kopfsegment zu den Nocken komplementäre Kerben vorgesehen sein.

Die Erfindung und Weiterbildungen der Erfindung werden im folgenden anhand der teilweise schematischen Darstellungen mehrerer Ausführungsbeispiele noch näher erläutert.

Es zeigen:
Fig. 1 eine Seitenansicht einer Ausführungsform des Längsträgers mit alternierend hintereinander angeordneten Längsträgerelementen vom Typ A und vom Typ B;
Fig. 2a einen Längsschnitt durch eine Ausführungsform der Vorrichtung, die nicht Teil der Erfindung ist;
Fig. 2b einen Querschnitt durch die in Fig. 2a dargestellte Ausführungsform der Vorrichtung; und
Fig. 3 einen Querschnitt durch eine Ausführungsform der erfindungsgemässen Vorrichtung.

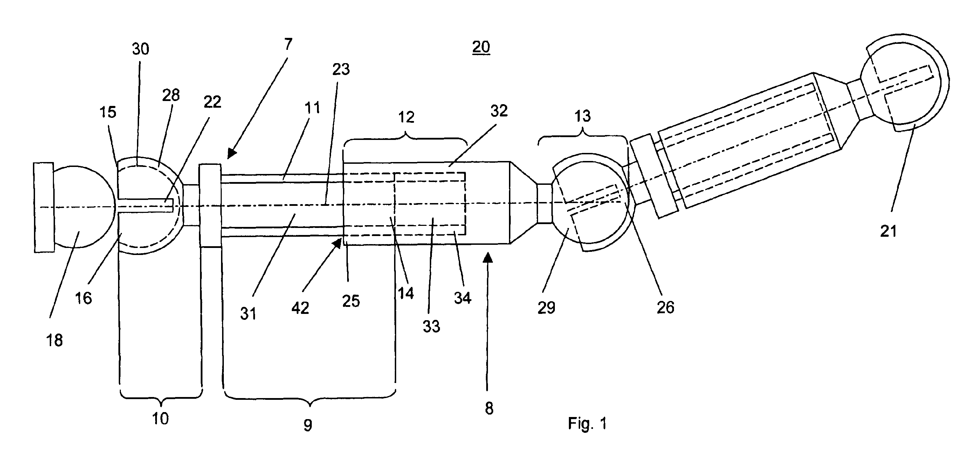

In Fig. 1 dargestellt ist eine Ausführungsform des Längsträgers 20 mit hintereinander alternierend angeordneten Längsträgerelementen 7;8 vom Typ A und vom Typ B und endständig je einem Endstück 18;21. Die Verbindungssegmente 9;10;12;13 der beiden Längsträgerelemente 7;8 sind derart ausgebildet, dass ein erstes Verbindungssegment 9 eines ersten Längsträgerelementes 7 (Typ A) mit einem ersten Verbindungssegment 12 eines zweiten Längsträgerelementes 8 (Typ B) eine koaxiale, nur axial längenverstellbare Verbindung bildet, während ein zweites Verbindungssegment 10 eines ersten Längsträgerelementes 7 (Typ A) mit einem zweiten Verbindungssegment 13 eines zweiten Längsträgerelementes 8 (Typ B) eine polyaxial schwenkbare Verbindung der zwei Längsträgerelemente 7;8 gestattet. Insbesondere sind hier die beiden ersten Verbindungssegmente 9;12 durch eine Gewindeverbindung 42 verbindbar, derart, dass die erste Längsachse 23 des Längsträgerelementes 7 (Typ A) und die zweite Längsachse 24 des Längsträgerelementes 8 (Typ B) koaxial angeordnet sind. Die Gewindeverbindung 42 ist hier derart ausgestaltet, dass das erste Verbindungssegment 9 des ersten Längsträgerelementes 7 (Typ A) einen Schaft 31 mit Aussengewinde 11 umfasst und das erste Verbindungssegment 12 des zweiten Längsträgerelementes 8 (Typ B) eine Hülse 32 mit einer ein zum Aussengewinde 11 komplementäres Innengewinde 34 aufweisende Zentralbohrung 33 umfasst. Die Ausgestaltung der Gewindeverbindung 42 könnte durch vertauschte Verbindungssegmente 9; 12 ausgebildet sein.

Das Längsträgerelement 7 (Typ A) umfasst zwei zur ersten Längsachse 23 koaxiale, hintereinander angeordnete Verbindungssegmente 9;10 und endständig ein erstes und ein zweites Ende 14;15. Dabei ist hier das erste Verbindungssegment 9 des Längsträgerelementes 7 (Typ A) als zylindrischer Schaft 31 mit einem Gewinde 11 ausgestaltet. Das zweite Verbindungssegment 10 des Längsträgerelementes 7 (Typ A) umfasst eine quer zur ersten Längsachse 23 elastisch deformierbare Gelenkschale 28 mit einer hohlkugelsegmentartigen, koaxialen Kavität 30, welche am zweiten Ende 15 des Längsträgerelementes 7 eine zur ersten Längsachse 23 konzentrische Öffnung 16 aufweist. Durch diese Öffnung 16 ist ein zur Kavität 30 komplementär ausgestalteter Gelenkkopf 29 koaxial in die Kavität 30 einführbar, so dass der Gelenkkopf 29 und die Gelenkschale 28 ein Kugelgelenk bilden. Die zwei so verbundenen Längsträgerelemente 7;8 (Typ A und B) sind dann derart relativ zueinander bewegbar, dass die erste und die zweite Längsachse 23;24 um das Gelenkzentrum relativ zueinander schwenkbar sind. Ferner weist die Kavität 30 einen Durchmesser D auf, der grösser als der Durchmesser d der Öffnung 16 ist, so dass ein in die Kavität 30 eingeführter Gelenkkopf 29 axial um mehr als 180° umschlossen wird. Die Gelenkschale 28 ist zudem mit vier zur Längsachse 23 parallelen Schlitzen 22 versehen, welche von aussen bis zur Kavität 30 durchgehend ausgebildet sind und eine radial elastische Deformation der Gelenkschale 28 gestatten.

Das Längsträgerelement 8 (Typ B) umfasst ebenfalls zwei koaxial zu seiner Längsachse hintereinander angeordnete Verbindungssegmente 12;13 sowie endständig ein erstes und ein zweites Ende 25;26. Das erste Verbindungssegment 12 des Längsträgerelementes 8 (Typ B) ist als zur zweiten Längsachse koaxiale Hülse 32 mit einer Zentralbohrung 33 ausgestaltet, wobei die Zentralbohrung 33 mit einem zum Gewinde 11 komplementären Innengewinde 34 versehen ist. Das zweite Verbindungssegment 13 des Längsträgerelementes 8 (Typ B) ist als sphärischer, zur Gelenkschale 28 komplementärer Gelenkkopf 29 ausgestaltet.

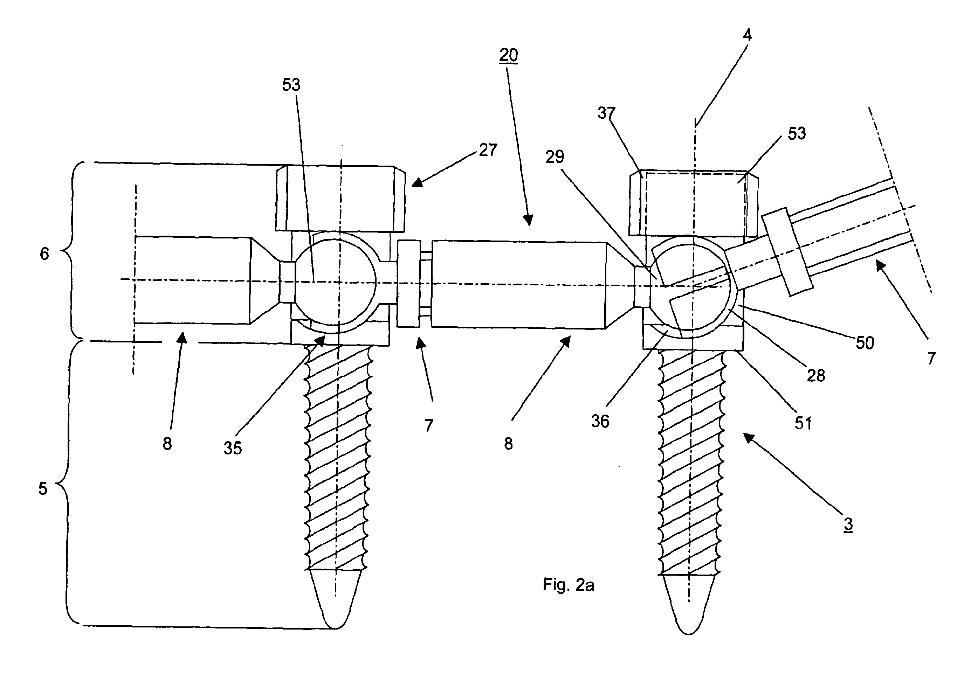

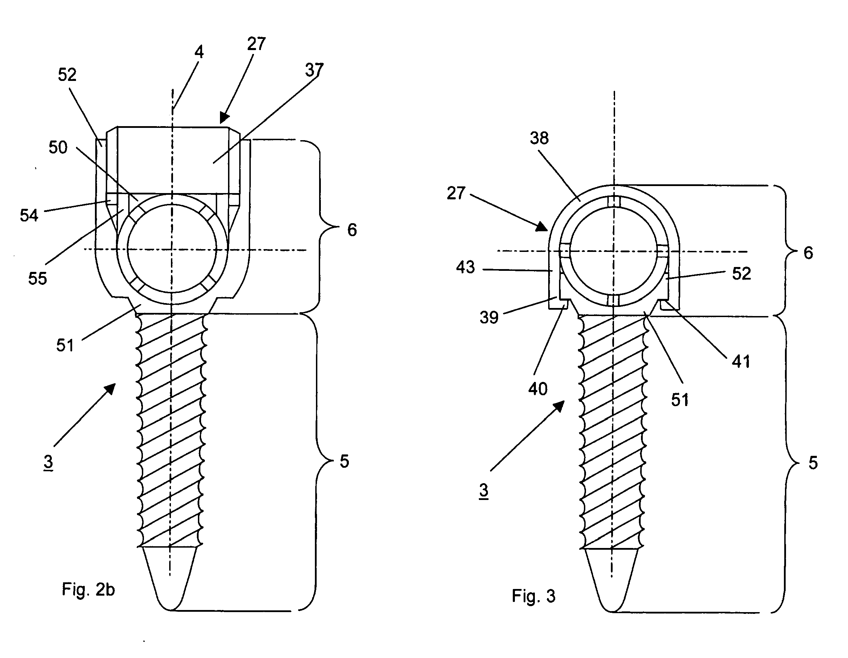

Das erste Endstück 18 ist analog zum Gelenkkopf 29 ausgebildet, während das zweite Endstück 21 analog zur Gelenkschale 28 ausgestaltet ist. Die zwei Endstücke 18;21 gestatten eine Aufnahme der Gelenkschale 28, respektive des Gelenkkopfes 29 beispielsweise in einer handelsüblichen Pedikelschraube oder Pedikelhaken mit einer sphärisch konkaven Ausnehmung 36 (Fig. 2).

In den Fig. 2a und 2b ist ein Ausschnitt aus einer Ausführungsform der erfindungsgemässen Vorrichtung 1 zur Stabilisierung von Wirbelkörpern mit einem Längsträger 20 gemäss Fig. 1 und zwei mit dem Längsträger 20 verbundenen Knochenverankerungsmitteln 3 dargestellt, wobei die Knochenverankerungsmittel 3 hier als Pedikelschrauben ausgebildet sind. Der Längsträger 20 umfasst mehrere alternierend angeordnete Längsträgerelemente 7;8 (Typ A und Typ B), wobei in dem hier dargestellten Ausschnitt zwei Längsträgerelemente 7 (Typ A) über ihre ersten Verbindungssegmente 9 mit den ersten Verbindungssegmenten 12 zweier Längsträgerelemente 8 (Typ B) gelenkig verbunden sind und mittig das Längsträgerelement 7 (Typ A) über sein zweites Verbindungssegment 10 mit dem Längsträgerelement 8 (Typ B) nur axial verschiebbar verbunden ist. Die Gelenkköpfe 29 der zweiten Längsträgerelemente 8 (Typ B) sind in die Gelenkschalen 28 der angrenzenden ersten Längsträgerelemente 7 (Typ A) eingeschnappt. Zur Verbindung der Kopfsegmente 6 der Knochenverankerungsmittel 3 mit den Gelenkschalen 29 sind an den Kopfsegmenten 6 Kanäle 50 mit quer zur Zentralachse 3 der Knochenverankerungsmittel 3 verlaufenden Kanalachsen 53 angeordnet. Die Kanäle 50 durchdringen die Kopfsegmente 6 der Knochenverankerungsmittel 3 quer zur Zentralachse 3 und sind am freien Ende 52 des Kopfsegmentes 6 offen. Zur polyaxial schwenkbaren Lagerung der Gelenkschalen 28 in den Kanälen 50 umfassen diese zu den Gelenkschalen 28 komplementär sphärische Ausnehmungen 36 an den Böden der Kanäle 50. Ferner umfassen die Kopfsegmente 6 vom freien Ende 52 her eindringende Bohrungen 55 mit Innengewinde 54. Die Fixationsmittel 27 zur Fixierung der Gelenkschalen 28 sind hier als Spannschrauben 37 ausgebildet, welche von den freien Enden 52 der Kopfsegmente 6 in die Innengewinde 54 schraubbar sind, so dass die in die Kanäle 50 eingelegten Gelenkschalen 28 zusammen mit den eingeschnappten Gelenkköpfen 29 in den Kanälen 50 durch Anziehen der Spannschrauben 37 fixierbar. Beim Fixieren der Gelenkschalen 28 werden diese quer zu den Längsachsen 23 der ersten Längsträgerelemente 7 (Typ A) zusammengepresst, wodurch die eingeschnappten Gelenkköpfe 29 der zweiten Längsträgerelemente 8 (Typ B) in den Gelenkschalen 28 blockiert werden.

Die in Fig. 3 dargestellte Ausführungsform der Vorrichtung 1 unterscheidet sich von der in den Fig. 2a und 2b dargestellten Ausführungsform nur darin, dass die Fixationsmittel 27 als Klammern 38 ausgebildet sind, deren Schenkel 43 elastisch spreizbar sind, so dass die Klammer 38 über die Gelenkschale 28 geführt werden kann und am Kopfsegment 6 des Knochenfixationsmittels 3 durch elastisches Zurückfedern einschnappbar ist, derart, dass die Gelenkschale 28 nach Montage durch die Klammer 38 zusammengepresst wird. Durch die Klammer 38 wird somit einmal die Gelenkschale 28 im Kopfsegment 6 des Knochenverankerungsmittels 3 festgehalten und zudem durch Zusammenpressen der Gelenkschale 28 der in der Gelenkschale 28 eingeschnappte Gelenkkopf 29 im Kopfsegment 6 des Knochenverankerungsmittels 3 blockiert. Für die Befestigung der Klammer 38 am Kopfsegment 6 umfasst die Klammer 38 an den freien Enden 39 der Schenkel 43 je einen nach innen gerichtete Nocken 40, welche in komplementäre, beim festen Ende 51 des Kopfsegmentes angeordnete einrastbar sind.

### Beschreibung des Operationsablaufes:

Zuerst werden vom Chirurgen die Knochenverankerungsmittel 3, insbesondere Pedikelschrauben oder Pedikelhaken an den Pedikeln der zu fixierenden Wirbelkörper gesetzt. Danach werden ausserhalb des Körpers des Patienten die Längsträgerelemente 7;8 (Typ A und Typ B) alternierend zusammengefügt bis der Längsträger 20 die gewünschte Länge aufweist und die gewünschte Anzahl Gelenke umfasst. Der zusammengesetzte Längsträger 20 wird dann durch eine minimal-invasive Inzision in den Körper des Patienten eingeführt und unter dem die zu fixierenden Wirbelkörper umgebenden Weichteilgewebe verschoben und an den Gelenken gebogen bis er die gewünschte Form aufweist und die Gelenkköpfe 29 in den Mitteln 35 zur Aufnahme der Gelenkköpfe 29 an den Knochenverankerungsmitteln 3 eingeführt sind. Anschliessend werden die Fixationsmittel 27 an den Kopfsegmenten 6 der Knochenverankerungsmittel 3 vormontiert, d.h. noch nicht blockiert, so dass der Längsträger 20 noch justierbar ist. In diesem Zustand sind die Form des Längsträgers 20 sowie die Abstände der einzelnen Längsträgerelemente 7;8 noch flexibel einstellbar.

Sobald die gewünschte Form des Längsträgers 20 eingestellt ist, werden die Fixationsmittel 27 blockiert und somit die gesamte Fixationsvorrichtung fixiert.

## Patentansprüche

1. Vorrichtung (1) zur Stabilisierung von Wirbelkörpern mit einem Längsträger (20) umfassend
A) ein erstes Längsträgerelement (7) mit einer Längsachse (23), zwei hintereinander angeordneten, koaxialen Verbindungssegmenten (9;10), einem ersten Ende (14) und einem zweiten Ende (15), wobei das erste Verbindungssegment (9) ein sich parallel zur Längsachse (23) erstreckendes Gewinde aufweist, wobei
A.1) das zweite Verbindungssegment (10) eine am zweiten Ende (15) des Längsträgerelementes (7) axial offene und quer zur Längsachse (23) elastisch deformierbare Gelenkschale (28) umfasst und
A.2) die Gelenkschale (28) eine hohlkugelsegmentartige Kavität (30) vom Durchmesser D mit einer am zweiten Ende (15) zur Längsachse (23) konzentrischen Öffnung (16) vom Durchmesser d umfasst, wobei d < D ist.
B) ein zweites Längsträgerelement (8) mit einer Längsachse wobei dieses zweite Längsträgerelement (8) ein erstes, mit dem ersten Verbindungssegment (9) des ersten Längsträgerelementes (7) verbindbares Verbindungssegment (12) und ein zweites Verbindungssegment (13) mit einem zur Gelenkschale (28) komplementären Gelenkkopf (29) umfasst;
C) mindestens zwei Knochenverankerungsmittel (3) mit je einer Zentralachse (4), je einem Verankerungssegment (5) zur Fixierung des Knochenverankerungsmittels (3) an einem Knochen, insbesondere an einem Wirbelkörper (2) und je einem Kopfsegment (6) mit Mitteln (35) zur Aufnahme der Gelenkschale (28); und
D) Fixationsmitteln (27) zur Fixierung der Gelenkschalen (28) in den Mitteln (35) zur Aufnahme der Gelenkschale (28),
**dadurch gekennzeichnet, dass**
E) die Fixationsmittel (27) eine elastisch spreizbare Klammer (38) umfassen, welche über die Gelenkschale (28) bringbar und am Kopfsegment (6) des Knochenfixationsmittels (3) einschnappbar ist, derart, dass
F) die Gelenkschale (28) nach Montage durch die Klammer (38) zusammengepresst wird.

2. Vorrichtung (1) nach Anspruch 1, **dadurch gekennzeichnet, dass** die Gelenkschale (28) aus Nitinol besteht.

3. Vorrichtung (1) nach Anspruch 1 oder 2, **dadurch gekennzeichnet, dass** die Gelenkschale (28) aussen sphärisch konvex ausgebildet ist.

4. Vorrichtung (1) nach einem der Ansprüche 1 bis 3, **dadurch gekennzeichnet, dass** das erste Verbindungssegment (9) ein zur Längsachse (23) koaxiales Aussengewinde (11) umfasst.

5. Vorrichtung (1) nach einem der Ansprüche 1 bis 4, **dadurch gekennzeichnet, dass** die Verbindung zwischen dem ersten Verbindungssegment (9) des ersten Längsträgerelementes (7) und dem ersten Verbindungssegment (12) des zweiten Längsträgerelementes (8) axial verlängerbar oder verkürzbar ist.

6. Vorrichtung (1) nach Anspruch 5, **dadurch gekennzeichnet, dass** die Verbindung zwischen dem ersten Verbindungssegment (9) des ersten Längsträgerelementes (7) und dem ersten Verbindungssegment (12) des zweiten Längsträgerelementes (8) teleskopierbar ist.

7. Vorrichtung (1) nach Anspruch 5, **dadurch gekennzeichnet, dass** die Verbindung zwischen dem ersten Verbindungssegment (9) des ersten Längsträgerelementes (7) und dem ersten Verbindungssegment (12) des zweiten Längsträgerelementes (8) eine Schraubverbindung ist.

8. Vorrichtung (1) nach einem der Ansprüche 1 bis 7, **dadurch gekennzeichnet**, der Gelenkkopf (29) sphärisch konvex und komplementär zur Kavität (30) ausgebildet ist.

9. Vorrichtung (1) nach einem der Ansprüche 1 bis 8, **dadurch gekennzeichnet, dass** der Gelenkkopf (29) und die Gelenkschale (28) elastisch ineinander einschnappbar sind.

10. Vorrichtung (1) nach einem der Ansprüche 7 bis 9, **dadurch gekennzeichnet, dass**
a) das erste Verbindungssegment (9) am ersten Längsträgerelementes (7) als Schaft (31) ausgebildet ist und das Gewinde als Aussengewinde (11) ausgestaltet ist; und
b) das erste Verbindungssegment (12) des zweiten Längsträgerelementes (8) als Hülse (32) ausgebildet ist, welche eine Zentralbohrung (33) mit einem zum Aussengewinde (11) komplementären Innengewinde (34) umfasst.

11. Vorrichtung (1) nach einem der Ansprüche 1 bis 10, **dadurch gekennzeichnet, dass** er mindestens ein Endstück (18) mit einem Gelenkkopf (29) umfasst.

12. Vorrichtung (1) nach einem der Ansprüche 1 bis 11, **dadurch gekennzeichnet, dass** er mindestens ein zweites Endstück (21) mit einer Gelenkschale (28) umfasst.

13. Vorrichtung (1) nach einem der Ansprüche 1 bis 12, **dadurch gekennzeichnet, dass** die Mittel (35) zur Aufnahme der Gelenkschale (28) eine konkave, zur Gelenkschale (28) komplementär sphärische Ausnehmung (36) umfassen.

14. Vorrichtung (1) nach einem der Ansprüche 1 bis 13, **dadurch gekennzeichnet, dass** die Klammer (38) an ihren freien Enden (39) je einen Nocken (40) umfasst, welcher am Kopfsegment (6) des Knochenverankerungsmittels (3) einrastbar ist.

15. Vorrichtung (1) nach Anspruch 14, **dadurch gekennzeichnet, dass** das Kopfsegment (6) zu den Nocken (40) komplementäre Kerben (41) umfasst.

## Claims

1. A device (1) to stabilise bodies of the vertebra with a longitudinal support (20) comprising
A) a first longitudinal support element (7) with a longitudinal axis (23), two coaxial connecting segments (9, 10) provided behind one another, with a first end (14) and a second end (15), wherein the first connecting segment (9) has a thread extending parallel to the longitudinal axis (23), wherein
A.1) on the second end (15) of the longitudinal support element (7) the second connecting segment (10) comprises a deformable articulated socket (28) that is axially open and can be deformed transversely to the longitudinal axis (23), and
A.2) the articulated socket (28) comprises a hollow spherical segment-like cavity (30) with a diameter of D with an opening (16) on the second end (15) that is concentric with the longitudinal axis (23) and has a diameter of d, while *d* < *D*;
B) a second longitudinal support element (8) with a longitudinal axis, wherein this second longitudinal support element (8) comprises a first connecting segment (12) that can be connected with the first connecting segment (9) of the first longitudinal support element (7) and a second connecting segment (13) with an articulated head (29) that is complementary to the articulated socket (28);
C) at least two bone anchoring means (3) each with a central axis (4), an anchoring segment (5) each to fix the bone anchoring means (3) on a bone, in particular on a body (2) of the vertebra and a head segment (6) each with means (35) to accommodate the articulated socket (28), and
D) fixing means (27) to fix the articulated sockets (28) in the means (35) to accommodate the articulated socket (28),
**characterized in that**
E) the fixing means (27) comprise an elastically expandable clamp (38), that can be placed over the articulated socket (28) and can be snapped onto the head segment (6) of the bone fixing means (3), in such a manner that
F) after the assembly the articulated socket (28) is compressed by the clamp (38).

2. The device (1) according to claim 1, **characterised in that** the articulated socket (28) is made from Nitinol.

3. The device (1) according to claim 1 or 2, **characterised in that** the articulated socket (28) has an external spherical convex construction.

4. The device (1) according to any one of claims 1 to 3, **characterised in that** the first connecting element (9) comprises an outside thread (11) that is coaxial with the longitudinal axis (23).

5. The device (1) according to any one of claims 1 to 4, **characterised in that** the connection between the first connecting segment (9) of the first longitudinal support element (7) and the first connecting segment (12) of the second longitudinal support element (8) can axially lengthened or shortened.

6. The device (1) according to claim 5, **characterised in that** the connection between the first connecting segment (9) of the first longitudinal support element (7) and the first connecting element (12) of the second longitudinal support element (8) can telescope.

7. The device (1) according to claim 5, **characterised in that** the connection between the first connecting segment (9) of the first longitudinal support element (7) and the first connecting element (12) of the second longitudinal support element (8) is a screw connection.

8. The device (1) according to any one of claims 1 to 7, **characterised in that** the articulated head (29) has a spherical convex construction that is complementary to the cavity (30).

9. A longitudinal support (20) according to any one of claims 1 to 8, **characterised in that** the articulated head (29) and the articulated socket (28) can be elastically snapped into one another.

10. The device (1) according to any one of claims 7 to 9, **characterised in that**
a) the first connecting segment (9) on the first longitudinal support element (7) is constructed as a shaft (31) and the thread is constructed as an outside thread (11), and
b) the first connecting segment (12) of the second longitudinal support element (8) is constructed as a sleeve (32), that comprises a central bore (33) with an inside thread (34) that complements the outside thread (11).

11. The device (1) according to any one of claims 1 to 10, **characterised in that** it comprises at least one end-piece (18) with an articulated head (29).

12. The device (1) according to any one of claims 1 to 11, **characterised in that** it comprises at least a second end-piece (21) with an articulated socket (28).

13. The device (1) according to any one of claims 1 to 12, **characterised in that** to accommodate the articulated socket (28) the means (15) comprise a concave spherical recess (36) that complements the articulated socket (28).

14. The device (1) according to any one of claims 1 to 13, **characterised in that** the clamp (38) has a lug (40) on each of its free end (39) that can be snapped onto the head segment (6) of the bone anchoring means (3).

15. The device (1) according to claim 14, **characterised in that** the head segment (6) comprises notches (41), complementing the lugs (40).

## Revendications

1. Dispositif (1) de stabilisation de corps vertébraux avec un support longitudinal (20), comprenant
A) un premier élément de support longitudinal (7) ayant un axe longitudinal (23), deux segments de liaison (9 ; 10) coaxiaux disposés l'un derrière l'autre, une première extrémité (14) et une deuxième extrémité (15), dans lequel le premier segment de liaison (9) présente un filetage s'étendant parallèlement à l'axe longitudinal (23), dans lequel
A.1) le deuxième segment de liaison (10) présente, au niveau de la deuxième extrémité (15) de l'élément de support longitudinal (7), une capsule d'articulation (28) ouverte axialement et déformable de manière élastique transversalement à l'axe longitudinal (23) et
A.2) la capsule d'articulation (28) comprend une cavité (30) en forme de segment de sphère creuse de diamètre D avec une ouverture (16) de diamètre d concentrique l'axe longitudinal (23) au niveau de la deuxième extrémité (15), où d < D,
B) un deuxième élément de support longitudinal (8) ayant un axe longitudinal, ce deuxième élément de support longitudinal (8) comprenant un premier segment de liaison (12) pouvant être relié au premier segment de liaison (9) du premier élément de support longitudinal (7) et un deuxième segment de liaison (13) présentant une tête d'articulation (29) complémentaire de la capsule d'articulation (28) ;
C) au moins deux moyens d'ancrage osseux (3) ayant chacun un axe central (4), chacun un segment d'ancrage (5) pour la fixation du moyen d'ancrage osseux (3) dans l'os, en particulier dans un corps vertébral (2), et chacun un segment de tête (6) présentant des moyens (35) pour loger la capsule d'articulation (28) ; et
D) des moyens de fixation (27) pour la fixation des capsules d'articulation (28) dans les moyens (35) pour loger la capsule d'articulation (28),
**caractérisé en ce que**
E) les moyens de fixation (27) comprennent une agrafe (38) pouvant être écartée de manière élastique, laquelle peut être placée sur la capsule d'articulation (28) et encliquetée au niveau du segment de tête (6) du moyen de fixation osseuse (3), de telle sorte que
F) la capsule d'articulation (28) est comprimée après le montage par l'agrafe (38).

2. Dispositif (1) selon la revendication 1, **caractérisé en ce que** la capsule d'articulation (28) est composée de Nitinol.

3. Dispositif (1) selon la revendication 1 ou 2, **caractérisé en ce que** la forme externe de la capsule d'articulation (28) est sphérique convexe.

4. Dispositif (1) selon l'une quelconque des revendications 1 à 3, **caractérisé en ce que** le premier segment de liaison (9) comprend un filetage (11) coaxial à l'axe longitudinal (23).

5. Dispositif (1) selon l'une quelconque des revendications 1 à 4, **caractérisé en ce que** la liaison entre le premier segment de liaison (9) du premier élément de support longitudinal (7) et le premier segment de liaison (12) du deuxième élément de support longitudinal (8) peut être rallongée ou raccourcie axialement.

6. Dispositif (1) selon la revendication 5, **caractérisé en ce que** la liaison entre le premier segment de liaison (9) du premier élément de support longitudinal (7) et le premier segment de liaison (12) du deuxième élément de support longitudinal (8) est télescopique.

7. Dispositif (1) selon la revendication 5, **caractérisé en ce que** la liaison entre le premier segment de liaison (9) du premier élément de support longitudinal (7) et le premier segment de liaison (12) du deuxième élément de support longitudinal (8) est une liaison par vis.

8. Dispositif (1) selon l'une quelconque des revendications 1 à 7, **caractérisé en ce que** la tête d'articulation (29) est conçue de manière sphérique convexe et complémentaire par rapport à la cavité (30).

9. Dispositif (1) selon l'une quelconque des revendications 1 à 8, **caractérisé en ce que** la tête d'articulation (29) et la capsule d'articulation (28) peuvent être emboîtées l'une dans l'autre de manière élastique.

10. Dispositif (1) selon l'une quelconque des revendications 7 à 9, **caractérisé en ce que**
a) le premier segment de liaison (9) est conçu, au niveau du premier élément de support longitudinal (7), sous forme de tige (31), et la vis est pourvue d'un filetage (11) ; et
b) le premier segment de liaison (12) du deuxième élément de support longitudinal (8) est conçu sous forme de douille (32), laquelle comprend un alésage central (33) avec un taraudage (34) complémentaire du filetage (11).

11. Dispositif (1) selon l'une quelconque des revendications 1 à 10, **caractérisé en ce qu'**il comprend au moins une pièce d'extrémité (18) avec une tête d'articulation (29).

12. Dispositif (1) selon l'une quelconque des revendications 1 à 11, **caractérisé en ce qu'**il comprend au moins une deuxième pièce d'extrémité (21) avec une capsule d'articulation (28).

13. Dispositif (1) selon l'une quelconque des revendications 1 à 12, **caractérisé en ce que** les moyens (35) pour loger la capsule d'articulation (28) comprennent un évidement (36) sphérique concave complémentaire de la capsule d'articulation (28).

14. Dispositif (1) selon l'une quelconque des revendications 1 à 13, **caractérisé en ce que** l'agrafe (38) comprend, au niveau de chacune de ses extrémités libres (39), un ergot (40) qui peut être encliqueté au niveau du segment de tête (6) du moyen d'ancrage osseux (3).

15. Dispositif (1) selon la revendication 14, **caractérisé en ce que** le segment de tête (6) comprend des encoches (41) complémentaires des ergots (40).
